# EUROPEAN PATENT SPECIFICATION

(11) **EP 0 578 723 B1**
(45) Date of publication and mention of the grant of the patent: **09.06.1999**
(21) Application number: 92908427.5
(22) Date of filing: 09.03.1992
(51) Int. Cl.: B01D 15/08, C07D 309/30

(54) **PROCESS FOR PURIFICATION OF HMG-CoA REDUCTASE INHIBITORS**
VERFAHREN ZUR REINIGUNG VON HMG-COA REDUCTASE-INHIBITOREN
PROCEDE DE PURIFICATION D'INHIBITEURS DE HMG-CoA REDUCTASE

(30) Priority: 13.03.1991 US 668831
(43) Date of publication of application: 19.01.1994
(73) Proprietor: Merck & Co., Inc., Rahway New Jersey 07065-0900 (US)
(72) Inventor: HAYTKO, Peter, N., Rahway, NJ 07065 (US); WILDMAN, Arthur, S., Jr., Martinsville, NJ 08836 (US)
(74) Representative: Quillin, Helen Kaye
(86) International application number: PCT/US92/01864
(87) International publication number: WO 92/16276

(56) References cited:
- US-A- 4 231 938
- US-A- 4 533 494
- US-A- 4 719 229
- US-A- 4 833 258
- US-A- 4 965 200
- US-A- 4 997 755
- US-A- 5 089 523
- US-A- 5 099 035
- US-A- 5 102 911
- THE MERCK INDEX, 11th Edition, Published 1989, (RAHWAY, NEW JERSEY), Compounds 5460, 7712, 8491.
- Book no. 5TH ED, 1973, 'CHEMICAL ENGINEERS' HANDBOOK', R.H. PERRY & C.H. CHILTON MCGRAW-HILL BOOK COMPANY, NEW YORK

## Description

### BACKGROUND OF THE INVENTION

High product purity is an important criterion for the manufacture of a safe and effective pharmaceutical. HMG-CoA reductase inhibitors, such as lovastatin, simvastatin and pravastatin, are a recently introduced new class of cholesterol-lowering agents.

The specific HMG-CoA reductase inhibitors lovastatin, mevastatin, pravastatin sodium and simvastatin are described in general terms in *The Merck Index*, 11th Edition, 1989, as entries 5460, 6088, 7712 and 8491 respectively.

US Patent 4,997,755 describes and claims a process for preparing 6-desmethyl-6β-hydroxymethyl and 6-desmethyl-6β-carboxy derivatives of lovastatin and analogues thereof by cultivation of a microorganism of the genus *Nocardia* (ATCC 53695).

US Patent 4,719,229, meanwhile, describes and claims a class of HMG-CoA reductase inhibitors structurally related to mevinolin which can be prepared by cultivating a strain of the microorganism *Aspergillus terreus*.

HMG-CoA reductase inhibitors effectively lower plasma cholesterol but must be taken on a long term basis. Thus it is particularly critical that HMG-CoA reductase inhibitors be administered in the highest possible purity.

Standard methods for the purification of organic molecules involve multiple recrystallization steps and employ large amounts of organic solvents. It would be highly desirable to employ a purification process that would yield a product purity of at least 99.5%, use no more than one crystallization with a recyclable solvent and be adaptable to high production volume.

High performance liquid chromatography (HPLC) is commonly used for the analytical determinations of compound purity. HPLC for large scale industrial solution preparations (preparative HPLC) has been employed in the separation and purification of proteins but it is believed not to have been employed in the large scale purification of relatively small molecules such as HMG-CoA reductase inhibitors.

### DETAILED DESCRIPTION OF THE INVENTION

This invention relates to a process for the purification of HMG-CoA reductase inhibitors by high performance liquid chromatography to yield a product of purity of at least 99.5%. The HMG-CoA reductase inhibitors within this invention include, but are not limited to, lovastatin, simvastatin, pravastatin, fluvastatin and mevastatin. The HPLC process of this invention offers a significant advantage in that no recrystallization is required to obtain a purity of at least 99.5% and typically only crystallization is employed. In addition, the HPLC process of this invention may be carried out with only one organic solvent, thus minimizing the need for recycling of solvent.

The process of this invention herein may employ either normal phase HPLC or reverse phase HPLC. For HMG-CoA reductase inhibitors exhibiting a tetrahydro-pyranone ring, such as lovastatin and simvastatin, the reverse phase procedure is preferred. The column packing may be uncoated silica, coated silica or porous graphitic carbon. The term coating as used herein includes both a physical and a chemical bonding of the binding group.

The crude HMG-CoA reductase inhibitor of approximately 85% or higher purity is dissolved in an organic solvent or a solution of an organic solvent and water. The mixture may be buffered to a pH between 2 and 9 with an organic or inorganic salt. Buffers may include, but are not limited to, Tris-acetate, or acetic acid/ammonia. The resulting solution is placed on an HPLC column. The column packing may be regular or irregular in shape. The diameter of the packing material may range from about 1 µm to about 100 µm. Preferably, the packing material is irregularly-shaped octadecylsilane and the diameter of the packing material is between about 3 µm and about 30 µm.

The column packings include, but are not limited to, silica, octylsilane, dimethylsilane, octadecylsilane, cyano-silane, or polystyrene-divinylbenzene copolymer with an organosilyl stationary phrase.

The column diameter may vary from 5 cm to 80 cm. The usual column length is approximately 25 cm. The column length may be extended as needed to effect the separation. Lengthening of the column may be accomplished by linking additional columns in series.

In general the column is packed with the coated or uncoated silica in the following manner: the packing material is slurried in ethanol. The slurry is then transferred into the column and compressed at 55 bar using Dynamic Axial Compression (D.A.C.*), a procedure described in U. S. Patent 3,996,609 and French Patent 73.07278. Alternatively, the column may be radially compressed. The ethanol is displaced with mobile phase. After packing the column is tested by collecting serial fractions and evaluating those fractions by standard analytical techniques.

The eluant is an organic solvent or a solution of an organic solvent and water which may also include a buffer of pH 2 to pH 9. The eluant is generally the same solvent or solvent mixture as the dissolving solvent but, if desired, the eluant may have a different composition. Preferably the eluant contains the same organic solvent and aqueous modifiers as the dissolving solvent. If desired, a gradient elution of the mobile phase may be employed to more rapidly elute the HMG-CoA reductase inhibitor through the column. The chromatography may be carried out at an operating temperature appropriate to the solvents employed, however a range of about 15° to about 60°C is preferred. In the preferred embodiment, isothermal conditions are maintained throughout the separation. Detection of the HMG-CoA reductase inhibitor may be by spectroscopic means or by other physical means such as optical rotation or refractive index. The preferred means are by ultraviolet absorption or refractive index. After the HMG-CoA reductase inhibitor peak of interest is collected, a portion of the solvent is removed and an aqueous solution is added to crystallize the HMG-CoA reductase inhibitor. Generally, about one-third of the solvent mixture is removed and water is employed to crystallize the HMG-CoA reductase inhibitor. Alternatively about two-thirds of the solvent mixture is removed to crystallize the HMG-CoA reductase inhibitor. The crystallized inhibitor is then filtered and dried to yield a product of purity of at least 99.5% and with an overall yield of about 90%. Product purity is determined by HPLC relative to a reference standard. Yield is determined by weight.

The crude HMG-CoA reductase inhibitor is prepared following any of the literature procedures well known to those skilled in this art. Packing materials of uncoated or coated silica are commercially available. Porous graphitic carbon as a packing material is also commercially available in pre-packed columns.

The organic solvent, employed as the dissolving solvent or the eluant, is selected from acetonitrile, methanol, ethanol, acetone, tetrahydrofuran, isopropanol, ethyl acetate, methylene chloride, chloroform or a mixture thereof. The percent of organic solvent in an organic solvent/water mixture may vary from about 10% to about 90% organic solvent, preferably 65% to 75% organic solvent.

If desired the amount of any residual solvent, particularly acetonitrile, may be decreased by dissolution of the purified HMG-CoA reductase inhibitor in aqueous methanol and crystallizing therefrom as shown below in Example 6.

### EXAMPLE 1

4.6 g of crude lovastatin was dissolved in 200 mL of 70:30 acetonitrile/water which was injected onto a 5 cm diameter, 25 cm long stainless steel column packed with 10 µm, irregular-shaped octadecylsilane HPLC packing material (RG1010-C18, The PQ Corporation, Conshohocken, PA). The eluant was 70:30 acetonitrile/water and the flow rate was approximately 150 mL/min. The lovastatin fraction was collected in a volume of 260 mL using UV detection at 254 nm. The lovastatin fraction was eluted at K' = 2.0-3.0. K', the capacity factor, is related to the retention time as described in USP - XXII (p. 1565; 1990). The resulting solution was concentrated by removal of one-third of the solvent, and the lovastatin was crystallized by the addition of water to give an acetonitrile concentration of approximately 25-30%. The pure lovastatin product was recovered by filtration and drying. Lovastatin with a purity of 99.7% w/w was recovered in an overall yield of 90%.

### EXAMPLE 2

Lovastatin at a concentration of 2.3 g/100 mL was dissolved in a mixture of 70% acetonitrile/30% 0.02 M Tris-acetate (pH 7.4). The solution was loaded onto a 5 cm diameter, 25 cm long stainless steel column packed with 10 µm, irregular-shaped octadecylsilane (RG1010-C18, The PQ Corporation, Conshohocken, PA). The eluant was 70% acetonitrile/30% water and the flow rate was approximately 150 mL/minute. Detection was by ultraviolet absorption at 254 nm. Lovastatin was eluted at K' = 2.0-3.0. The lovastatin peak was collected and one third the volume was removed by vacuum distillation at ≤ 40°C. Water was added to bring the acetonitrile concentration to 25-30%. The lovastatin was filtered and dried in vacuo at ≤ 40°C. Lovastatin with a purity of ≥ 99.7% was recovered in an overall yield of ≥ 90%.

### EXAMPLE 3

4.6 g of crude lovastatin was dissolved in 200 mL of 70:30 acetonitrile/water buffered with 0.02 M Tris-acetate (pH 7.5). The solution was loaded onto a 5 cm diameter, 25 cm long column packed with 10 µm, irregular-Shaped octadecylsilane HPLC packing (RG1010-C18, The PQ Corporation, Conshohocken, PA). The eluant was 70:30 acetonitrile/water and the flow rate was approximately 150 mL/min. The lovastatin fraction was collected in a volume of 265 mL using UV detection at 254 nm. The lovastatin peak eluted at K' = 2.0-3.0. The resulting solution was concentrated by removal of one third of the solvent. Lovastatin was crystallized by the addition of water to give an acetonitrile concentration of approximately 25-30%. The pure lovastatin product was recovered by filtration and drying. Lovastatin with a purity of 99.7% w/w was recovered in an overall yield of 91%.

### EXAMPLE 4

4.6 g of crude lovastatin was dissolved in 200 mL of 70:30 acetonitrile/water buffered with 0.02 M Tris-acetate (pH 7.5). The solution was loaded onto a 5 cm diameter, 25 cm long column packed with 10 µm, irregular-shaped octadecylsilane HPLC packing (RG1010-C18, The PQ Corpodration, Conshohocken, PA). The eluant was 70:30 acetonitrile/water and the flow rate was approximately 150 mL/min. The lovastatin fraction was collected in a volume of 265 mL using UV detection at 254 nm. The lovastatin peak eluted at K' = 2.0-3.0. The resulting solution was concentrated by removal of two-thirds of the solvent, which crystallized the lovastatin. The pure lovastatin product was recovered by filtration and drying. Lovastatin with a purity of 99.7% w/w was recovered in an overall yield of 91%.

### EXAMPLE 5

Lovastatin at a concentration of 4.5 g/100 mL was dissolved in a mixture of 70% acetonitrile/30% 0.02 M Tris-acetate (pH 7.2). The 40°C solution was injected onto a 5 cm diameter, 25 cm long stainless steel column packed with 10-20 µm, irregular-shaped octadecylsilane HPLC packing (RG1020-C18, The PQ Corporation, Conshohocken, PA). The eluant was 70:30 acetonitrile/water and the flow rate was approximately 150 mL/min. The media and column were isothermally maintained at 40°C. The lovastatin fraction was collected in a volume of 500 mL using UV detection at 254 nm. The lovastatin fraction eluted at K' = 2.0-3.0. The resulting solution was concentrated by removal of two-thirds of the solvent and the lovastatin crystallized. The lovastatin was recovered by filtration and drying. Lovastatin with a purity 99.8% w/w was recovered in an overall yield of 90%.

### EXAMPLE 6

6.0 g of the purified lovastatin prepared as described in Example 5 was dissolved in 100 mL of 95% methanol/5% water at 60° C. The 60° C solution was crystallized by the addition of an equal volume of 65% water/35% methanol. The resulting crystalline mixture was concentrated to one half volume. Lovastatin was recovered by filtration and drying. 5.98 g of lovastatin was recovered.

### EXAMPLE 7

Simvastatin may be purified to a crystalline form of purity greater than 99.5% using procedures analogous to those described in Example 5. Crude simvastatin is used in place of crude lovastatin.

### EXAMPLE 8

Pravastatin may be purified to a crystalline form of purity greater than 99.5% using a procedure analogous to that in Example 5, but substituting crude pravastatin for the crude lovastatin.

## Claims

1. A process for the large scale industrial purification of a crude HMG-CoA reductase inhibitor which comprises:
(1) placing a solution of the crude HMG-CoA reductase inhibitor on a high performance liquid chromatography column wherein said column is packed with silica optionally coated with a stationary phase selected from the group consisting of a triorganosilyl, a cyanoorganosilyl and a polystyrene-divinylbenzene copolymer with an organosilyl, or said column is packed with a porous graphitic carbon;
(2) eluting a fraction containing HMG-CoA reductase inhibitor with a solvent mixture comprising:
(a) an organic solvent selected from the group consisting of acetonitrile, methanol, ethanol, acetone, tetrahydrofuran, isopropanol, ethyl acetate, methylene chloride or chloroform, or a mixture thereof and optionally
(b) water or an aqueous solution selected from the group consisting of phosphoric acid and acetic acid;
(3) removing about 30 to 35 percent of the solvent mixture from the eluted fraction containing HMG-CoA reductase inhibitor; and
(4) treating the eluted fraction containing HMG-CoA reductase inhibitor fraction with water to crystallize the HMG-CoA reductase inhibitor, and
(5) filtering and drying the HMG-CoA reductase inhibitor to yield a product of purity greater than or equal to 99.5%.

2. The process of Claim 1 wherein the HMG-CoA reductase inhibitor is selected from the group consisting of lovastatin, simvastatin, pravastatin, fluvastatin or mevastatin.

3. The process of Claim 2 wherein the HMG-CoA reductase inhibitor is selected from lovastatin or simvastatin.

4. The process of Claim 3 wherein the HMG-CoA reductase inhibitor is lovastatin.

5. The process of any one of the preceding claims wherein the column is packed with a silica coated with an octadecylsilane stationary phase.

6. The process of any one of the preceding claims wherein the solvent mixture is acetonitrile and water.

7. The process of Claim 6 wherein the solvent mixture is 70% acetonitrile and 30% water.

8. The process of any one of the preceding claims wherein the operating temperature of the chromatography is between 15° to 60°C.

9. The process of any one of the preceding claims wherein about one-third of the solvent mixture is removed from the eluted fraction containing the HMG-CoA reductase inhibitor.

10. A process for the large scale industrial purification of a crude HMG-CoA reductase inhibitor which comprises:
(1) placing a solution of the crude HMG-CoA reductase inhibitor on a high performance liquid chromatography column wherein said column is packed with silica optionally coated with a stationary phase selected from the group consisting of a triorganosilyl, a cyanoorganosilyl and a polystyrene-divinylbenzene copolymer with an organosilyl, or said column is packed with a porous graphitic carbon;
(2) eluting a fraction containing HMG-CoA reductase inhibitor with a solvent mixture comprising:
(a) an organic solvent selected from the group consisting of acetonitrile, methanol, ethanol, acetone, tetrahydrofuran, isopropanol, ethyl acetate, methylene chloride or chloroform or a mixture thereof and optionally
(b) water or an aqueous solution selected from the group consisting of phosphoric acid and acetic acid; and
(3) removing about 60 to 65% of the solvent mixture from the eluted fraction containing HMG-CoA reductase inhibitor to crystallize the HMG-CoA reductase inhibitor, and
(4) filtering and doing the HMG-CoA reductase inhibitor to yield a product of purity greater than or equal to 99.5%.

## Patentansprüche

1. Ein Verfahren für die industrielle Reinigung eines rohen HMG-CoA-Reduktase-Inhibitors im großen Umfang, das umfaßt:
(1) Aufbringen einer Losung des rohen HMG-CoA-Reduktase-Inhibitors auf eine Hochleistungsflüssigchromatographie-Säule, wobei die Säule mit Silica gepackt ist, das gegebenenfalle mit einer stationären Phase beschichtet ist, die ausgewählt ist aus der Gruppe, die aus einem Triorganosilyl, einem Cyanoorganosilyl und einem Polystyrol-Divinylbenzol-Copolymer mit einem Organosilyl besteht, oder wobei die Säule mit einem porösen graphitischen Kohlenstoff gepackt ist;
(2) Eluieren einer Fraktion, die HMG-CoA-Reduktase-Inhibitor enthält, mit einem Lösungsmittelgemisch, das enthält:
(a) ein organisches Lösungsmittel, das ausgewählt ist aus der Gruppe, die aus Acetonitril, Methanol, Ethanol, Aceton, Tetrahydrofuran, Isopropanol, Ethylacetat, Methylenchlorid oder Chloroform oder einer Mischung davon besteht, und gegebenenfalls
(b) Wasser oder eine wäßrige Lösung, die ausgewählt ist aus der Gruppe, die aus Phosphorsäure und Essigsäure besteht;
(3) Entfernen von etwa 30 bis 35% des Lösungsmittelgemischs aus der eluierten Fraktion, die HMG-CoA-Reduktase-Inhibitor enthält; und
(4) Behandeln der eluierten Fraktion, die HMG-CoA-Reduktase-Inhibitor-Fraktion enthält, mit Wasser, um den HMG-CoA-Reduktase-Inhibitor zu kristallisieren, und
(5) Filtrieren und Trocknen des HMG-CoA-Reduktase-Inhibitore, um ein Produkt mit einer Reinheit von größer als oder gleich 99,5% zu erhalten.

2. Das Verfahren nach Anspruch 1, wobei der HMG-CoA-Reduktase-Inhibitor ausgewählt ist aus der Gruppe, die aus Lovastatin, Simvastatin, Pravastatin, Fluvastatin oder Mevastatin besteht.

3. Das Verfahren nach Anspruch 2, wobei der HMG-CoA-Reduktase-Inhibitor ausgewählt ist aus Lovastatin oder Simvastatin.

4. Das Verfahren nach Anspruch 3, wobei der HMG-CoA-Reduktase-Inhibitor Lovastatin ist.

5. Das Verfahren nach irgendeinem der vorangehenden Ansprüche, wobei die Säule mit einem Silica gepackt ist, das mit einer stationären Octadecylcilan-Phase beschichtet ist.

6. Das Verfahren nach irgendeinem der vorangehenden Ansprüche, wobei das Lösungsmittelgemisch Acetonitril und Wasser ist.

7. Das Verfahren nach Anspruch 6, wobei das Lösungsmittelgemisch 70% Acetonitril und 30% Wasser ist.

8. Das verfahren nach irgendeinem der vorangehenden Ansprüche, wobei die Arbeitstemperatur der Chromotographie zwischen 15° bis 60°C ist.

9. Das verfahren nach irgendeinem der vorangehenden Ansprüche, wobei etwa ein Drittel des Lösungsmittelgemischs aus der eluierten Fraktion, die den HMG-CoA-Reduktase-Inhibitor enthält, entfernt wird.

10. Ein Verfahren für die industrielle Reinigung eines rohen HMG-CoA-Reduktase-Inhibitors im großen Umfang, das umfaßt:
(1) Aufbringen einer Lösung des rohen HMG-CoA-Reduktase-Inhibitors auf eine Hochleistungsflüssigchromatographie-Säule, wobei die Säule mit Silica gepackt ist, das gegebenenfalls mit einer stationären Phase beschichtet ist, die ausgewählt ist aus der Gruppe, die aus einem Triorganosilyl, einem Cyanoorganosilyl und einem Polystyrol-Divinylbenzol-Copolymer mit einem Organosilyl besteht, oder wobei die Säule mit einem, porösen graphitischen Kohlenstoff gepackt ist,
(2) Eluieren einer Fraktion, die HMG-CoA-Reduktase-Inhibitor enthält, mit einem Lösungsmittelgemisch, das enthält:
(a) ein organisches Lösungsmittel, das ausgewählt ist aus der Gruppe, die aus Acetonitril, Methanol, Ethanol, Aceton, Tetrahydrofuran, Isopropanol, Ethylacetat, Methylenchlorid oder Chloroform oder einer Mischung davon besteht, und gegebenenfalls
(b) Wasser oder eine wäßrige Lösung, die ausgewählt ist aus der Gruppe, die aus Phosphorsäure und Essigsäure besteht; und
(3) Entfernen von etwa 60 bis 65% des Lösungsmittelgemischs aus der eluierten Fraktion, die HMG-CoA-Reduktase-Inhibitor enthält, um den HMG-CoA-Reduktase-Inhibitor zu kristallisieren; und
(4) Filtrieren und Trocknen des HMG-CoA-Reduktase-Inhibitors, um ein Produkt mit einer Reinheit von größer als oder gleich 99,5% zu erhalten.

## Revendications

1. Procédé de purification industrielle à grande échelle d'un inhibiteur de HMG-CoA réductase brut qui comprend les étapes consistant à:
(1) placer une solution de l'inhibiteur de HMG-CoA réductase brut sur une colonne de chromatographie liquide haute performance, ladite colonne étant garnie de silice éventuellement enrobée d'une phase stationnaire choisie dans le groupe constitué par un triorganosilyle, un cyanoorganosilyle et un copolymère polystyrène/divinylbenzène avec un organosilyle, ou ladite colonne étant garnie d'un carbone graphitique poreux;
(2) éluer une fraction contenant l'inhibiteur de HMG-CoA réductase avec un mélange de solvants comprenant:
(a) un solvant organique choisi dans le groupe constitué par l'acétonitrile, le méthanol, l'éthanol, l'acétone, le tétrahydrofurane, l'isopropanol, l'acétate d'éthyle, le chlorure de méthylène ou le chloroforme, ou un mélange de ceux-ci et, le cas échéant,
(b) de l'eau ou une solution aqueuse choisie dans le groupe constitué par l'acide phosphorique et l'acide acétique;
(3) éliminer environ 30 à 35 pour cent du mélange de solvants de la fraction éluée contenant l'inhibiteur de HMG-CoA réductase; et
(4) traiter la fraction éluée contenant l'inhibiteur de HMG-CoA réductase avec de l'eau pour cristalliser l'inhibiteur de HMG-CoA réductase; et
(5) filtrer et sécher l'inhibiteur de HMG-CoA réductase pour obtenir un produit de pureté supérieure ou égale à 99,5%.

2. Procédé selon la revendication 1, dans lequel l'inhibiteur de HMG-CoA réductase est choisi dans le groupe constitué par la lovastatine, la simvastatine, la pravastatine, la fluvastatine ou la mévastatine.

3. Procédé selon la revendication 2, dans lequel l'inhibiteur de HMG-CoA réductase est choisi parmi la lovastatine ou la simvastatine.

4. Procédé selon la revendication 3, dans lequel l'inhibiteur de HMG-CoA réductase est la lovastatine.

5. Procédé selon l'une quelconque des revendications précédentes, dans lequel la colonne est garnie d'une silice enrobée d'une phase stationnaire octadécylsilane.

6. Procédé selon l'une quelconque des revendications précédentes, dans lequel le mélange de solvants est de l'acétonitrile et de l'eau.

7. Procédé selon la revendication 6, dans lequel le mélange de solvants est 70% d'acétonitrile et 30% d'eau.

8. Procédé selon l'une quelconque des revendications précédentes, dans lequel la température de fonctionnement de la chromatographie est comprise entre 15° et 60°C.

9. Procédé selon l'une quelconque des revendications précédentes, dans lequel environ le tiers du mélange de solvants est éliminé de la fraction éluée contenant l'inhibiteur de HMG-CoA réductase.

10. Procédé de purification industrielle à grande échelle d'un inhibiteur de HMG-CoA réductase brut qui comprend les étapes consistant à:
(1) placer une solution de l'inhibiteur de HMG-CoA réductase brut sur une colonne de chromatographie liquide haute performance, ladite colonne étant garnie de silice éventuellement enrobée d'une phase stationnaire choisie dans le groupe constitué par un triorganosilyle, un cyanoorganosilyle et un copolymère polystyrène/divinylbenzène avec un organosilyle, ou ladite colonne étant garnie d'un carbone graphitique poreux;
(2) éluer une fraction contenant l'inhibiteur de HMG-CoA réductase avec un mélange de solvants comprenant:
(c) un solvant organique choisi dans le groupe constitué par l'acétonitrile, le méthanol, l'éthanol, l'acétone, le tétrahydrofurane, l'isopropanol, l'acétate d'éthyle, le chlorure de méthylène ou le chloroforme, ou un mélange de ceux-ci et, le cas échéant,
(d) de l'eau ou une solution aqueuse choisie dans le groupe constitué par l'acide phosphorique et l'acide acétique; et
(3) éliminer environ 60 à 65% du mélange de solvants de la fraction éluée contenant l'inhibiteur de HMG-CoA réductase pour cristalliser l'inhibiteur de HMG-CoA réductase; et
(4) filtrer et sécher l'inhibiteur de HMG-CoA réductase pour obtenir un produit de pureté supérieure ou égale à 99,5%.
